# EUROPÄISCHE PATENTSCHRIFT

(11) **EP 3 755 226 B1**
(45) Veröffentlichungstag und Bekanntmachung des Hinweises auf die Patenterteilung: **27.12.2023**
(21) Anmeldenummer: 20727182.6
(22) Anmeldetag: 13.05.2020
(51) Int. Cl.: A61B 5/16, A61B 5/00

(54) **SYSTEM UND VERFAHREN ZUR ERKENNUNG UND MESSUNG AFFEKTIVER ZUSTÄNDE**
SYSTEM AND METHOD FOR RECOGNISING AND MEASURING AFFECTIVE STATES
SYSTÈME ET PROCÉDÉ DE DÉTECTION ET DE MESURE D'ÉTATS AFFECTIFS

(30) Priorität: 16.05.2019 DE 102019113002
(43) Veröffentlichungstag der Anmeldung: 30.12.2020
(73) Patentinhaber: Tawny GmbH, 80799 München (DE)
(72) Erfinder: MAIER, Marco, 81373 München (DE); BARTL, Michael, 80639 München (DE)
(74) Vertreter: Prüfer & Partner mbB Patentanwälte · Rechtsanwälte
(86) Internationale Anmeldenummer: PCT/EP2020/063393
(87) Internationale Veröffentlichungsnummer: WO 2020/229572

(56) Entgegenhaltungen:
- US-A1- 2012 052 476
- Fernando Bevilacqua: "GAME-CALIBRATED AND USER-TAILORED REMOTE DETECTION OF EMOTIONS", , 1. Januar 2018 (2018-01-01), XP55716001, ISBN: 978-91-9-841879-8 Gefunden im Internet: URL:http://his.diva-portal.org/smash/get/d iva2:1259426/FULLTEXT01.pdf [gefunden am 2020-07-17]
- MOHAMMED E HOQUE ET AL: "Robust Recognition of Emotion from Speech", 1. Januar 2006 (2006-01-01), INTELLIGENT VIRTUAL AGENTS LECTURE NOTES IN COMPUTER SCIENCE;LECTURE NOTES IN ARTIFICIAL INTELLIG ENCE;LNCS, SPRINGER, BERLIN, DE, PAGE(S) 42 - 53, XP019039024, ISBN: 978-3-540-37593-7 Zusammenfassung; Abbildung 1
- GUTHIER BENJAMIN ET AL: "Affective Computing in Games", 6. Oktober 2016 (2016-10-06), PERVASIVE: INTERNATIONAL CONFERENCE ON PERVASIVE COMPUTING; [LECTURE NOTES IN COMPUTER SCIENCE; LECT.NOTES COMPUTER], SPRINGER, BERLIN, HEIDELBERG, PAGE(S) 402 - 441, XP047358179, ISBN: 978-3-642-17318-9 [gefunden am 2016-10-06] Abschnitt 4.1 "Evaluating Player Experience", zweiter Absatz Abschnitt "5 Conclusions", vierter Absatz
- RICCARDO BERTA ET AL: "Electroencephalogram and Physiological Signal Analysis for Assessing Flow in Games", IEEE TRANSACTIONS ON COMPUTATIONAL INTELLIGENCE AND AI IN GAMES, IEEE, USA, vol. 5, no. 2, 1 June 2013 (2013-06-01), pages 164-175, XP011514677, ISSN: 1943-068X, DOI: 10.1109/TCIAIG.2013.2260340
- MALLAPRANDA, CHANDANA: "Classification of EEG signals of user states in gaming using machine learning. Masters Thesis. Missouri University of science and technology", N/A, 12 December 2018 (2018-12-12), XP009535042,

## Beschreibung

Die Erfindung betrifft ein Verfahren zur Erkennung affektiver Zustände eines Subjekts. Die Erfindung betrifft ferner ein Verfahren zur Einrichtung eines Systems zur Erkennung affektiver Zustände. Insbesondere betrifft die Erfindung die Erkennung affektiver Zustände mittels physiologischer Signale.

Das Forschungsfeld *"Affective Computing"* (im Folgenden "AC") beschäftigt sich mit dem Erkennen, Verarbeiten, Interpretieren und Simulieren von menschlichen Affekten und Emotionen. Im Hinblick auf das Ziel, Emotionen zu erkennen, basieren typische Ansätze auf verschiedenen Arten von Sensordaten wie Bildern, Videos, Audiodaten und physiologischen Signalen wie Herzfrequenz (HR) oder elektrodermale Aktivität (EDA). Die so erfassten oder erkannten Affekte können dann beispielsweise zur Steuerung von Computersystemen oder Umgebungsvariablen eines Benutzers verwendet werden, wie die stimmungsabhängige Anpassung der Umgebungsbeleuchtung oder -temperatur.

Neben grundlegenden Emotionen wie *Freude, Angst, Trauer, Wut oder Überraschung* können auch andere psychologische Modelle wie die Flow-Theorie ein wertvolles Konstrukt sein, um den affektiven Zustand eines Benutzers zu beurteilen. Der Flow-Zustand zeichnet sich durch optimale Erfahrung, vollständigen Eintauchen und hohe Produktivität aus und macht ihn zu einer interessanten Information bei der Beurteilung von Benutzererfahrungen, Mediennutzung, Arbeitsproduktivität, oder generell von Überforderungs- bzw. Unterforderungszuständen im Rahmen der Ausübung von Tätigkeiten.

Traditionell wird durch Fragebögen bestimmt, ob ein Subjekt einen Flow erlebt oder nicht, was den Nachteil hat, dass der Flow-Zustand erst nach dessen tatsächlichem Auftreten erfassbar ist und vom Subjekt händische Arbeit erfordert. Die Messung durch Fragebögen unterliegt zudem dem Bias einer häufig verzerrten Selbsteinschätzung und -bewertung.

Es ist daher Aufgabe der Erfindung, Verfahren bereitzustellen, die der Nachteil aus dem Stand der Technik beseitigen und eine Möglichkeit bereitstellen, eine implizite Flow-Messung zu ermöglichen und zu automatisieren

Diese Aufgabe wird mit einem Verfahren zur Einrichtung eines Systems zur Erkennung affektiver Zustände eines Subjekts nach Anspruch 1 gelöst.

Vorteilhafte Weiterbildungen sind Gegenstand der Unteransprüche.

Offenbart wird ein Verfahren zur Einrichtung eines Systems zur Erkennung affektiver Zustände eines Subjekts, das die Schritte aufweist: a. Bereitstellen einer Umgebung, die eingerichtet ist, ein Trainingssubjekt in eine Gruppe von affektiven Zuständen zu versetzen, wobei die Gruppe von affektiven Zuständen mindestens einen ersten affektiven Zustand und einen zweiten affektiven Zustand umfasst und der erste affektive Zustand und der zweite affektive Zustand unterschiedlich sind, b. Bereitstellen eines Systems zur Einrichtung eines Systems zur Erkennung affektiver Zustände, wobei das System ein lernendes System ist und eine erste Eingabevorrichtung zur Eingabe von physiologischen Informationen über ein Trainingssubjekts und eine zweite Eingabevorrichtung zur Eingabe oder automatischen Erfassung des Vorliegens eines affektiven Zustands des Trainingssubjekts aufweist, c. Versetzen des Trainingssubjektes in einen affektiven Zustand aus der Gruppe affektiver Zustände, d. Erfassung der physiologischer Informationen über das Trainingssubjekt, e. Ermittlung des affektiven Zustands, f. Speicherung der erfassten physiologischen Information über das Trainingssubjekt unter Zuordnung des ermittelten affektiven Zustands, g. Eingabe der erfassten physiologischen Information in die erste Eingabevorrichtung, h. Eingabe des ermittelten affektiven Zustands in die zweite Eingabevorrichtung, und i. Verarbeitung der Eingabe in der ersten Eingabevorrichtung und in der zweiten Eingabevorrichtung zum Training des Systems zur Erkennung affektiver Zustände. Hierbei kann die Eingabe über die erste Eingabevorrichtung und die zweite Eingabevorrichtung auch dadurch erfolgen, dass die Eingabe über einen einheitlichen Datenblock, beispielsweise als Zuführen einer Dateien an das System erfolgt, wobei die jeweiligen Daten in einem internen Prozess separiert werden und dann unterschiedlichen Verarbeitungsvorgängen unterworfen werden. Ferner muss hierbei das Versetzen in einen affektiven Zustands des Trainingssubjekts nicht notwendigerweise durch das System oder das Verfahren selbst erfolgen. Hier ist auch eine externe Stimulanz oder der affektive Zustand, den das Trainingssubjekt mitbringt, ausreichend. Erforderlich ist lediglich, dass sich das Trainingssubjekt beim Training in einem definierten affektiven Zustand befindet. Ferner kann die Gruppe aus den mindestens zwei affektiven Zuständen auch aus einem affektiven Zustand und dessen Komplement beispielsweise verängstigt oder angstfrei bzw. gestresst oder stressfrei bestehen.

Offenbart wird das Verfahren zur Einrichtung eines Systems zur Erkennung affektiver Zustände eines Subjekts, wobei die Schritte c. bis i. für mehrere Trainingssubjekte oder für mehrere affektive Zustände widerholt werden.

Offenbart wird das Verfahren zur Einrichtung eines Systems zur Erkennung affektiver Zustände eines Subjekts, wobei die Gruppe affektiver Zustände den affektiven Zustand Langeweile/Unterforderung, den affektiven Zustand Flow und den affektiven Zustand Stress/Überforderung umfasst.

Offenbart wird das Verfahren zur Einrichtung eines Systems zur Erkennung affektiver Zustände eines Subjekts, wobei die physiologischen Informationen visuelle Informationen, physiologische Signalinformationen oder akustische Informationen sind.

Offenbart wird das Verfahren zur Einrichtung eines Systems zur Erkennung affektiver Zustände eines Subjekts, wobei die visuellen Informationen Einzelbildinformation oder Bewegtbildinformationen eines menschlichen Gesichts sind, die physiologischen Signalinformationen elektrodermale Aktivitätsinformationen, Herzfrequenzinformationen und Herzfrequenzvarianzinformationen sind, oder die akustischen Informationen die Aufnahme einer menschlichen Stimme ist.

Offenbart wird das Verfahren zur Einrichtung eines Systems zur Erkennung affektiver Zustände eines Subjekts, wobei die Umgebung, die eingerichtet ist, das Trainingssubjekt in eine Gruppe von affektiven Zuständen zu versetzen, eine Aufgabenstellvorrichtung, beispielsweise eine elektronische Datenverarbeitungsvorrichtung umfasst, mittels der das Trainingssubjekt eine Aufgabe gestellt wird, beispielsweise das Spiel Tetris^{®} zu spielen, wobei die Aufgabe so eingerichtet ist, dass sie mindestens so viele Schwierigkeitsstufen aufweist, wie es affektive Zustände in der Gruppe der affektiven Zustände gibt, und es eine objektive Zuordnung von Schwierigkeitsstufen zu den affektiven Zuständen in der Form gibt, dass das Trainingssubjekt Lösen der Aufgabe in einer der Schwierigkeitsstufen in einen bestimmten affektiven Zustand versetzt wird.

Offenbart wird das Verfahren zur Einrichtung eines Systems zur Erkennung affektiver Zustände eines Subjekts, wobei das Spiel Tetris^{®} die Schwierigkeitsstufen leicht, mittel und schwer aufweist, wobei die Schwierigkeitsstufen so eingerichtet sind, dass sie das Trainingssubjekt beim Spielen der jeweiligen Schwierigkeitsstufe in den Zustand Langeweile, Flow, bzw. Stress versetzen.

Offenbart wird das Verfahren zur Einrichtung eines Systems zur Erkennung affektiver Zustände eines Subjekts, wobei das lernende System ein neuronales Netz, ein faltendes neuronales Netz (CNN) oder ein rekurrentes neuronales Netz (RNN) aufweist.

Offenbart wird das Verfahren zur Einrichtung eines Systems zur Erkennung affektiver Zustände eines Subjekts, wobei das lernende System ein faltendes neuronales Netz aufweist, das aus vier Faltungsschichten mit 32 Filtern und einer Kernelgröße von 3 besteht, die Schichten über Max-Pooling-Schichten verbunden sind, nach den Konvolutionen eine vollständig verbundene Schicht (fully connected layer) mit 32 Neuronen zu einer abschließenden dichten Schicht (dense layer) führt, und die abschließende dichte Schicht eine Anzahl von Neuronen entsprechend der Anzahl der Klassen der Klassifikationsaufgabe und einer Softmax-Aktivierung aufweist.

Offenbart wird ein System zur Einrichtung eines Systems zur Erkennung affektiver Zustände eines Subjekts, das aufweist: eine Umgebung, die eingerichtet ist, ein Trainingssubjekt in eine Gruppe von affektiven Zuständen zu versetzen, wobei die Gruppe von affektiven Zuständen mindestens einen ersten affektiven Zustand und einen zweiten affektiven Zustand umfasst und der erste affektive Zustand und der zweite affektive Zustand unterschiedlich sind, eine erste Eingabevorrichtung zur Eingabe von physiologischen Informationen über ein Trainingssubjekts und eine zweite Eingabevorrichtung zur Eingabe des Vorliegens eines affektiven Zustands des Trainingssubjekts aufweist, wobei das System ein lernendes System ist und eingerichtet und bestimmt ist das Verfahren zur Einrichtung eines Systems zur Erkennung affektiver Zustände eines Subjekts durchzuführen.

Offenbart wird das System zur Einrichtung eines Systems zur Erkennung affektiver Zustände eines Subjekts, wobei die Umgebung, die eingerichtet ist, das Trainingssubjekt in eine Gruppe von affektiven Zuständen zu versetzen, eine elektronische Datenverarbeitungsvorrichtung umfasst, mittels der das Trainingssubjekt das Spiel Tetris^{®} zu spielen, wobei das Spiel so eingerichtet ist, dass es so viele Schwierigkeitsstufen aufweist, wie es affektive Zustände in der Gruppe der affektiven Zustände gibt, und es eine objektive Zuordnung von Schwierigkeitsstufen zu den affektiven Zuständen in der Form gibt, dass das Trainingssubjekt beim Spielen des Spiels Tetris^{®} in einer der Schwierigkeitsstufen in einen bestimmten affektiven Zustand versetzt wird.

Offenbart wird das System zur Einrichtung eines Systems zur Erkennung affektiver Zustände eines Subjekts, wobei das Spiel Tetris^{®} die Schwierigkeitsstufen leicht, mittel und schwer aufweist, wobei die Schwierigkeitsstufen so eingerichtet sind, dass sie das Trainingssubjekt beim Spielen der jeweiligen Schwierigkeitsstufe in den Zustand Langeweile, Flow, bzw. Stress versetzen.

Offenbart wird das System zur Einrichtung eines Systems zur Erkennung affektiver Zustände eines Subjekts, wobei das lernende System ein neuronales Netz, ein faltendes neuronales Netz (CNN) oder ein rekurrentes neuronales Netz (RNN) aufweist.

Offenbart wird das System zur Einrichtung eines Systems zur Erkennung affektiver Zustände eines Subjekts, wobei das lernende System ein faltendes neuronales Netz aufweist, das aus vier Faltungsschichten mit 32 Filtern und einer Kernelgröße von 3 besteht, die Schichten über Max-Pooling-Schichten verbunden sind, nach den Konvolutionen eine vollständig verbundene Schicht (fully connected layer) mit 32 Neuronen zu einer abschließenden dichten Schicht (dense layer) führt, und die abschließende dichte Schicht eine Anzahl von Neuronen entsprechend der Anzahl der Klassen der Klassifikationsaufgabe und einer Softmax-Aktivierung aufweist.

Offenbart wird das System zur Einrichtung eines Systems zur Erkennung affektiver Zustände eines Subjekts, wobei die erste Eingabevorrichtung zur Eingabe von physiologischen Informationen über ein Trainingssubjekts mit einer Kamera zur Aufnahme von Bewegtbildinformation eines Gesichts des Trainingssubjekts, einem Armbandgerät, das physiologische Signale wie elektrodermale Aktivität, Herzfrequenz oder Herzfrequenzvariabilität erfasst, oder einem Mikrofon zur Erfassung einer Stimme des Trainingssubjekts verbunden ist.

Offenbart wird ein System zur Erkennung affektiver Zustände eines Subjekts, wobei das System lernend ist und nach dem Verfahren zur Einrichtung eines Systems zur Erkennung affektiver Zustände eines Subjekts gemäß einem der vorhergehenden Absätze trainiert wurde.

Offenbart wird ein Verfahren zur Erkennung affektiver Zustände eines Subjekts, das die Schritte aufweist: a. Bereitstellen eines Systems nach dem vorhergehenden Anspruch, mit einer ersten Eingabevorrichtung zur Eingabe von physiologischen Informationen über ein Subjekt und einer ersten Ausgabevorrichtung zur Ausgabe des Vorliegens eines affektiven Zustands des Subjekts, b. Erfassung und Eingabe der physiologischen Informationen über das Subjekt in die erste Eingabevorrichtung, c. Verarbeitung der Eingabe der physiologischen Information durch das System, d. Klassifikation eines affektiven Zustands des Subjekts durch das System, e. Ausgabe des klassifizierten affektiven Zustands des Subjekts über die erste Ausgabe.

Offenbart wird das Verfahren zur Erkennung affektiver Zustände eines Subjekts nach einem der vorhergehenden Absätze, wobei der klassifizierte affektive Zustand des Subjekts einer aus Langeweile, Flow und Stress ist.

Offenbart wird das Verfahren zur Erkennung affektiver Zustände eines Subjekts nach einem der vorhergehenden Absätze, wobei die physiologischen Informationen visuelle Informationen, physiologische Signalinformationen oder akustische Informationen sind.

Offenbart wird das Verfahren zur Erkennung affektiver Zustände eines Subjekts nach dem vorhergehenden Absatz, wobei die visuellen Informationen Bewegtbildinformationen eines menschlichen Gesichts sind, die physiologischen Signalinformationen elektrodermale Aktivitätsinformationen, Herzfrequenzinformationen und Herzfrequenzvarianzinformationen sind, oder die akustischen Informationen die Aufnahme einer menschlichen Stimme ist.

Offenbart wird ein System zur Erkennung affektiver Zustände eines Subjekts, das eingerichtet ist, eines der vorhergehenden Verfahren zur Erkennung affektiver Zustände eines Subjekts auszuführen, das aufweist: das System zur Erkennung affektiver Zustände eines Subjekts nach einem der vorhergehenden Absätze, die erste Eingabevorrichtung zur Eingabe von physiologischen Informationen über das Subjekts und einer ersten Ausgabevorrichtung zur Ausgabe des Vorliegens eines affektiven Zustands des Subjekts.

Offenbart wird ein System zur Erkennung affektiver Zustände eines Subjekts nach einem der vorhergehenden Ansprüche, wobei die erste Eingabevorrichtung eine Kamera zur Aufnahme von Bewegtbildern, ein Armbandgerät, das physiologische Signale wie elektrodermale Aktivität, Herzfrequenz oder Herzfrequenzvariabilität erfasst, oder ein Mikrofon zur Sprachaufzeichnung umfasst.

Offenbart wird das System zur Erkennung affektiver Zustände eines Subjekts nach einem der vorhergehenden Absätze, das einen zentralen Dienst aufweist, wobei der zentrale Dienst in mindestens folgende Schichten aufgeteilt ist: eine Interaktionsschicht, die so eingerichtet ist, dass Benutzer zur Erkennung affektiver Zustände des Subjekts physiologische Informationen an das System übermitteln können, eine Auswertungsschicht, die eingerichtet ist, aus den übermittelten physiologischen Informationen mittels des Verfahren zur Einrichtung eines Systems zur Erkennung affektiver Zustände eines Subjekts nach einem der vorhergehenden Ansprüche trainierten lernenden Systems den affektiven Zustand des Subjekts ermittelt, und eine Datenbasisschicht, die Trainingsdaten zum Trainieren des lernenden Systems speichert.

Offenbart wird das System zur Erkennung affektiver Zustände eines Subjekts nach einem der vorhergehenden Absätze, wobei die Interaktionsschicht dem Benutzer eine über ein Weitverkehrsnetz zugängliche Benutzungsschnittstelle bereitstellt, über die Dateien mit physiologischen Informationen an das System übermittelt werden können und über die dem Benutzer Informationen über den aus den physiologischen Informationen zugeordnete affektive Zustand angezeigt oder dem Benutzer als übertragbare Datei bereitgestellt werden.

Offenbart wird das System zur Erkennung affektiver Zustände eines Subjekts nach einem der vorhergehenden Absätze, wobei das System eingerichtet ist, die Interaktionsschicht einem Ermittlungsbenutzer die über das Weitverkehrsnetz zugängliche Benutzungsschnittstelle bereitzustellen, über die der Ermittlungsbenutzer Inhalte bereitstellen kann, die von einem Zielbenutzer konsumiert werden, die Interaktionsschicht dem Zielbenutzer zu ermöglichen, während des Konsums erfasste physiologische Informationen an das System zu übermitteln, und dem Ermittlungsbenutzer Informationen über den aus den physiologischen Informationen zugeordnete affektive Zustand anzuzeigen oder dem Ermittlungsbenutzer als übertragbare Datei bereitzustellen.

Offenbart wird das System zur Erkennung affektiver Zustände eines Subjekts nach einem der vorhergehenden Absätze, wobei die Auswertungsschicht in mehrere Module unterteilt ist, ein visuelles Modul eingerichtet ist, aus visuellen Informationen, insbesondere Bewegtbildinformationen eines menschlichen Gesichts als physiologischen Informationen den affektiven Zustand des Subjekts zu ermittelt, ein physiologisches Modul eingerichtet ist, aus physiologischen Signalinformationen, insbesondere elektrodermale Aktivitätsinformationen, Herzfrequenzinformationen und Herzfrequenzvarianzinformationen als physiologische Information den affektiven Zustand des Subjekts zu ermittelt, und ein akustisches Modul eingerichtet ist, aus akustischen Informationen, insbesondere der Aufnahme einer menschlichen Stimme als physiologische Information den affektiven Zustand des Subjekts zu ermittelt.

Zusammenfassung: Der "Flow" genannte affektive Zustand ist als Zustand optimaler Erfahrung, vollständiger Vertiefung und hoher Produktivität definiert. Als wichtige Metrik für verschiedene Szenarien, die von (Profi-) Sport über Arbeitswelten bis hin zu Benutzererfahrungsauswertung (User Experience Evaluationen) bei der Softwareentwicklung reichen, wurde er üblicherweise mit Hilfe traditioneller Fragebögen umfassend untersucht. Um die Flow-messung für Online-Echtzeitumgebungen zugänglich zu machen, stellt diese Erfindung die Ergebnisse zur automatischen Bestimmung des Flow-Zustandes eines Benutzers basierend auf physiologischen Signalen vor, die mit einem tragbaren Gerät gemessen oder über eine Bildaufzeichnung (Bewegt- oder Einzelbild) erfasst werden. Hierbei wird eine Studie mit Zielpersonen durchgeführt, die das Spiel Tetris^{®} in verschiedenen Schwierigkeitsgraden spielen, was zu Langeweile, Stress und Flow führt. Mit einem CNN ("Convolutional Neural Network", "faltendes neuronales Netz") wird eine Genauigkeit von 70% bei der Erkennung Flow-induzierenden Werten erreicht. Als mögliche zukünftige Entwicklung wird erwartet, dass Flow ein potenzielles Belohnungssignal für Human-in-the-Loop-Lernsysteme sein kann.

Einführung: Das Forschungsfeld *"Affective Computing"* (im Folgenden "AC") beschäftigt sich mit dem Erkennen, Verarbeiten, Interpretieren und Simulieren von menschlichen Affekten und Emotionen. Im Hinblick auf das Ziel, Emotionen zu erkennen, basieren typische Ansätze auf verschiedenen Arten von Sensordaten wie Bildern, Videos, Audiodaten und physiologischen Signalen wie Herzfrequenz (HR) oder elektrodermale Aktivität (EDA). Neben grundlegenden Emotionen wie *Freude, Angst, Trauer, Wut oder Überraschung* können auch andere psychologische Modelle wie die Flow-Theorie ein wertvolles Konstrukt sein, um den affektiven Zustand eines Benutzers zu beurteilen. Der Flow-Zustand zeichnet sich durch optimale Erfahrung, vollständigen Eintauchen und hohe Produktivität aus und macht ihn zu einer interessanten Information bei der Beurteilung von Benutzererfahrungen bezüglich Benutzungsoberflächen von Spiele bis hin zu ganzen Umgebungen. Traditionell wird durch Fragebögen bestimmt, ob ein Subjekt einen Flow erlebt oder nicht, was den Nachteil hat, dass der Flow-Zustand erst nach dessen tatsächlichem Auftreten erfassbar ist und vom Subjekt händische Arbeit erfordert. Im Gegensatz dazu ist eine automatische Flow-Erkennung auf Basis von Sensordaten implizit, unauffällig und in Echtzeit möglich.

Die Erfindung offenbart ein Verfahren zur automatischen Messung des Flows mit physiologischen Signalen von beispielsweise am Handgelenk getragenen Geräten. Das Verfahren basiert auf einer CNN-Architektur (Convolutional Neural Network). Für die Generierung von Trainingsdaten wird ein Studienaufbau mit dem bekannten Spiel Tetris^{®} vorgestellt. Ergebnisse werden anhand einer Pilotstudie offenbart.

Studienaufbau: Für die Datenerfassung liegt eine modifizierte Version des Spiels Tetris^{®} als mobile Anwendung vor. Tetris^{®} wurde bereits in ähnlichen Studien eingesetzt und es wurde festgestellt, dass je nach Schwierigkeitsgrad des Spiels, die Benutzer Flow erfahren. Die ursprüngliche Spiellogik wurde so modifiziert, dass es nur drei verschiedene Stufen gibt, nämlich *leicht, normal* und *schwer,* in zufälliger Reihenfolge, jeweils 10 Minuten lang und unabhängig von der Leistung des Spielers. Die Schwierigkeit der drei Stufen, d.h. die Geschwindigkeit der fallenden Tetriminos, wurde so festgelegt, wie vorab erwartet wurde, dass das Spiel zu *Langeweile, Fluss* und *Stress* führen würde. Die aufgezeichneten physiologischen Daten aus jeder Ebene wurden entsprechend gekennzeichnet.

Die Teilnehmer wurden so ausgewählt, dass sie alle ungefähr das gleiche Leistungsniveau im Spiel hatten. Sie waren mit einem Empatica E4 Armbandgerät ausgestattet, das physiologische Signale wie elektrodermale Aktivität (im Folgenden EDA), Herzfrequenz (im Folgenden HR) und Herzfrequenzvariabilität (im Folgenden HRV) erfasst. Das E4-Gerät wurde an der nicht dominanten Hand des Teilnehmers getragen. Das Smartphone (iPhone 5s) mit der Tetris^{®}-Anwendung wurde in der anderen (dominanten) Hand gehalten.

Die folgenden vorläufigen Auswertungen wurden an einem Datensatz aus einer kleinen Pilotstudie durchgeführt. Es gab elf Teilnehmer (drei weiblich, acht männlich) im Alter zwischen 20 und 35 Jahren. Insgesamt haben wir 31 Sitzungen gesammelt, die bis zu 15,5 Stunden an Daten ansammelten. Vier Teilnehmer spielten mehrere Sessions, sieben Teilnehmer spielten nur eine Session.

Daten und Vorbereitung: Drei Ströme von physiologischen Signalen aus dem E4 wurden verwendet: HR, HRV und EDA. HR und EDA werden von der E4 bereitgestellt und wurden in ihrer Rohform verwendet. Für die HRV stellt die E4 die sogenannten RR-Intervalle zur Verfügung, d.h. die Zeitdifferenz zwischen aufeinanderfolgenden Herzschlägen, aus der verschiedene HRV-Messungen abgeleitet werden können. Die EDA wird mit 4 Hz abgetastet, während die HR-Werte mit 1 Hz angegeben werden. RR-Intervalle werden nicht in regelmäßigen Abständen bereitgestellt, sondern wenn sie auftreten. Um die RR-Intervalle mit den beiden anderen Datenströmen in Einklang zu bringen, wurde ein gemeinsames HRV-Maß namens RMSSD ("root mean square of successive differences", "quadratischer Mittelwert aus aufeinanderfolgenden Differenzen") berechnet.

**Tabelle 1: Beste mittlere Testgenauigkeiten bei der Kreuzvalidierung bei Auslassung einer Session (leave-one-session-out) und Auslassung eines Teilnehmers (leave-one-subject-out).**

| Genauigkeit (%) | Basislinie | Auslassung einer Session | Auslassung eines Teilnehmers |
|---|---|---|---|
| Langeweile vs. nicht Langeweile | 50.00 | 65.04 | 57.13 |
| Flow vs. Nicht-Flow | 50.00 | 70.37 | 69.55 |
| Stress vs. Nicht-Stress | 50.00 | 66.09 | 71.17 |
| Langeweile vs. Flow vs. Stress | 33.33 | 52.59 | 50.43 |

Das RMSSD-Maß wird über Datenbereiche berechnet und es wird empfohlen, eine Bereichsgröße von mindestens 60 Sekunden zu verwenden. Folglich wurde bei jedem Zeitschritt, bei dem ein RR-Wert empfangen wurde, ein Bereich der Größe 60 Sekunden vor diesem Zeitpunkt extrahiert und der RMSSD-Wert für diesen Bereich berechnet. Die Stichprobenzeiten der EDA-Serie wurden als Grundlage für die letzte Zeitreihe verwendet. Sowohl HR- als auch RMSSD-Werte wurden vorwärtsgefüllt, um der 4 Hz Abtastfrequenz der EDA-Serie zu entsprechen. Das Ergebnis ist eine äquidistante Zeitreihe, die mit 4 Hz abgetastet wird, mit EDA-, HR- und HRV-Werten (d.h. RMSSD) zu jedem Zeitschritt.

Um die Trainings- und Validierungssets zu erstellen, wurde jede Sitzung in Bereiche mit *n* Proben aufgeteilt. Das Bereichsintervall wird für eine Probe nach der anderen verschoben, d.h. aufeinanderfolgende Bereiche überlappen sich um *n* 1 Proben. 10-Sekundenbereiche wurden verwendet, d.h. Bereiche, die aus *n* = 40 Samples bestehen, die jeweils drei Werte enthalten. Die Bereichslänge von 10 Sekunden wurde gewählt, da erste Tests zeigten, dass kürzere Bereiche es nicht erlauben, die charakteristischen Muster zu erfassen.

Training und Evaluierung: Der Ansatz basiert auf einer CNN-Architektur. Das Netz besteht aus vier Faltungsschichten (32 Filter, Kernelgröße 3), die über Max-Pooling-Schichten verbunden sind. Nach den Konvolutionen führt eine vollständig verbundene Schicht (fully connected layer, 32 Neuronen) zu einer abschließenden dichten Schicht (dense layer) mit der Anzahl der Neuronen entsprechend der Anzahl der Klassen der Klassifikationsaufgabe und einer Softmax-Aktivierung. Bis auf die letzte Schicht wurden ReLU-Aktivierungen für die Schichten verwendet. Während des Trainings wird der Dropout nach der Faltungsschicht (0,1) und der dichten Schicht (0,5) angewendet, um eine Überanpassung (overfitting) zu vermeiden.

Drei binäre One-Vs-all-Klassifizierungsaufgaben wurden evaluiert und eine Aufgabe, die versuchte, zwischen allen drei Klassen gleichzeitig zu unterscheiden. Bei der Erstellung der Trainings- und Validierungsdatensätze wurden die Beispiele ausgewogen ausgewählt, d.h. für die binären Aufgaben wurde nur die Hälfte der Beispiele für die negative Klasse zufällig aus den verfügbaren Beispielen gezogen, um eine gleichmäßige Aufteilung zwischen den beiden Klassen zu gewährleisten. Wir haben unser Modell auf zwei Arten trainiert und bewertet: leave-one-session-out Kreuzvalidierung und leave-one-subject-out Kreuzvalidierung, letztere nur bei Probanden, die nur eine Sitzung gespielt hatten, also Validierung bei einem völlig unbekannten Probanden in jeder Wiederholung. Tabelle 1 zeigt die Ergebnisse.

Man kann sehen, dass die Beispiele, die mit Langeweile verbunden sind, am schwersten richtig einzuschätzen sind. Es wird angenommen, dass die einfache Ebene unter den drei Ebenen zu der höchsten Diversität an Gefühlen führt, d.h. die sehr langsame Geschwindigkeit wird manchmal als entspannend, manchmal als stressig und nur manchmal als ausgesprochen langweilig empfunden. Insgesamt ist das CNN-Modell in der Lage, zwischen den drei Klassen deutlich genauer zu unterscheiden als die Basisstrategie.

Wie bereits erwähnt, ist der affektive Flow-Zustand oft mit einer hohen Produktivität oder einer besseren Leistung verbunden. Im vorliegenden Fall kann die erzielte Punktzahl im Tetris^{®}-Spiel als die Produktivität des Benutzers interpretiert werden. So kann man das Modell auf Tetris^{®}-Sitzungen anwenden, um eine Sitzung in Intervalle von Langeweile, Flow und Stress zu unterteilen - diesmal ohne Rücksicht auf Informationen über den tatsächlichen Spielstand - und dann beobachten, wie gut die Leistung des Spielers in den jeweiligen Zuständen ist. Die Spieler schnitten in der Tat am besten ab, wenn das Modell den Flow-Zustand erkannt hat (durchschnittlich 2,59 Punkte pro 10-Sekunden-Fenster), am zweitbesten, wenn der Spieler als gelangweilt eingeschätzt wird (2,04 Punkte). Im Gegensatz dazu, wenn unser System den Zustand des Stresses erkennt, schneiden die Spieler deutlich schlechter ab und erhalten in diesen Phasen sogar negative Werte (-0,50 Punkte).

Aus psychologischer Sicht sollte weiter überprüft werden, ob die affektiven Zustände, die mit den verschiedenen Schwierigkeitsgraden des Spiels versucht wird zu induzieren, wirklich als *Langeweile, Stress* und *Flow* angesehen werden können. Obwohl der allgemeiner Aufbau mit früheren Studien zur Untersuchung des Flusses und insbesondere des Flusses mit dem Spiel Tetris^{®} übereinstimmt, wurde die genaue Spielvariante und die Umgebungsbedingungen nicht vollständig überprüft. Daher ist es ratsam, den Datenerhebungsprozess mit psychologisch validierten Flow-Fragebögen zu kombinieren.

Andererseits konnte beobachtet werden, dass die Spieler in den Zeitintervallen, die das Modell als Flow einstuft, am besten abschneiden, was als Indikator für ein tatsächliches Flow-Erlebnis angesehen werden kann.

Alles in allem eröffnen die positiven ersten Ergebnisse mehrere Möglichkeiten für die weitere Arbeit. Neben der Verbesserung des Datensatzes und der Anpassung des Modells gibt es viel Potenzial in der Übertragung des allgemeinen Ansatzes auf andere, ähnliche Aufgaben, insbesondere typische Aufgaben eines Bürojobs.

Deutlicher auf den Bereich der KI-Forschung ausgerichtet, ist besonders interessant, die automatische Flow-Erkennung als Feedback-Mechanismus beim Human-in-the-Loop-Verstärkungslernen zu nutzen. Sozial intelligente Agenten könnten von den Informationen über diesen affektiven Zustand profitieren, indem sie ihn als Belohnungssignal für ihr Verhalten integrieren.

Offenbart wird ein zentraler Dienst mit mehreren Schichten. Die oberste Schicht eine Interaktionsschicht dar. Der Nutzer kann Video-, Audio- oder biometrische Daten hochladen und erhält die Emotionsanalyse seiner Inhalte in einem Online-Dashboard oder optional als Rohdaten-Download (z. B. csv-Datei) zur individuellen Weiterverarbeitung zurück. Alternativ können Benutzer andere Teilnehmer einladen, Anwendungen in Echtzeit über die Plattform zu testen. Gesichtsemotionen, Sprache, tragbare Daten werden aufgezeichnet, während der Konsum digitaler Inhalte (z. B. Video, Werbung, Produktpräsentation usw.) konsumiert oder die Durchführung einer Testaufgabe (z. B. App, Website UX/UI, Interview, Chatbot, Spiel usw.) durchführt.

Die mittlere Schicht ist eine Auswertungsschicht. Die Plattform wurde entwickelt, um menschliche Daten wie Mimik, Sprache oder biometrische Daten von tragbaren Sensoren wie Herzfrequenzvariabilität (HRV), Herzfrequenz (HR), elektrodermale Aktivität der Haut (EDA), Hauttemperatur, Photoplethysmographie (Blutvolumenpuls) und Bewegung (Beschleunigungssensor, Gyroskop) vorzuverarbeiten. Die Verarbeitung kann einzeln oder in verschiedenen Kombinationen durchgeführt werden. Neuronale Netzwerke und Deep-Learning-Verfahren sind in die verteilte und Echtzeit-Softwarearchitektur eingebettet, um Muster für die Klassifizierung menschlicher Zustände wie emotionalen Stress, Glück, Aufmerksamkeit, Produktivitätsniveau, Fluss usw. zu identifizieren. Die Klassifizierung menschlicher Affekte wird durch aus der Psychophysiologie und Sozialwissenschaft bekannte Inventare, Skalen und Tests unterstützt (z. B. PANAS-Skala, Oxford Glückskatalog, Flow-Konstrukt etc.). Ein visuelles Modul, ein physiologisches Modul und ein akustisches Modul sind die Out-of-the-Box-Kernmodule, die zusätzlich zur Integration in andere (Software-)Produkte und kundenspezifische Unternehmenslösungen auf Basis von Lizenzgebühren angeboten werden können.

Die Basisschicht bildet eine Datenbasisschicht, mit dem maschinelle Lernmodelle wie Convolutional Neural Networks (CNN) und Recurrent Neural Networks (RNN) trainiert werden, um menschliche emotionale Bedingungen basierend auf visuellen, akustischen und physiologischen Daten zu klassifizieren. Diese proprietären Daten ermöglichten es, eine Lernumgebung für emotionale Klassifikationsmodelle zu schaffen.

Im Folgenden wird die Erfindung anhand eines Ausführungsbeispiels und den Figuren erläutert.
Figur 1 zeigt ein System zur Einrichtung eines Systems zur Erkennung affektiver Zustände eines Subjekts.
Figur 2 zeigt ein Ablaufdiagramm eines erfindungsgemäßen Verfahrens zur Einrichtung eines Systems zur Erkennung affektiver Zustände eines Subjekts.
Figur 3 zeigt ein System zur Erkennung affektiver Zustände eines Subjekts.
Figur 4 zeigt ein Ablaufdiagramm eines erfindungsgemäßen Verfahrens zur Erkennung affektiver Zustände eines Subjekts.

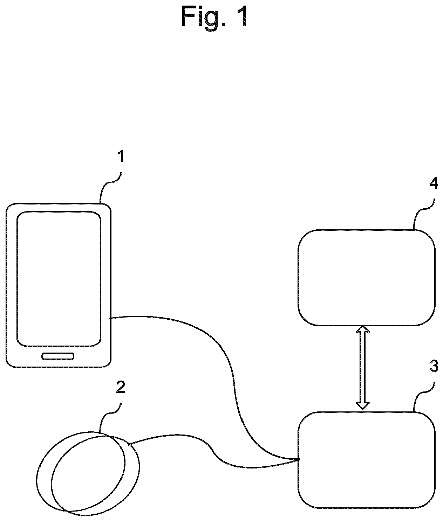

Anhand von Figur 1 wird ein System zur Einrichtung eines Systems zur Erkennung affektiver Zustände eines Subjekts beschrieben.

Das Einrichtungssystem weist eine Affektzustandserzeugungsvorrichtung 2 auf. Die Affektzustandserzeugungsvorrichtung 2 ist im Ausführungsbeispiel eine elektronische Datenverarbeitungsvorrichtung, insbesondere eine mobile Datenverarbeitungsvorrichtung wie ein Smartphone oder ein Tablet, auf dem eine modifizierte Form des Computerspiels Tetris^{®} installiert ist. Das Spiel Tetris^{®} ist so modifiziert, dass es drei Schwierigkeitsstufen leicht, normal und schwierig aufweist. Die Schwierigkeitsstufen sind so ausgestaltet, dass ein durchschnittlicher Spieler beim Spielen von Tetris^{®} in der Schwierigkeitsstufe leicht den affektiven Zustand "Langeweile", in der Schwierigkeitsstufe mittel den affektiven Zustand "Flow" und in der Schwierigkeitsstufe schwer den affektiven Zustand "Stress" erfährt.

Der Versuchsaufbau mit der modifizierten Form des Computerspiels Tetris^{®} ist hier nur ein möglicher Auslöser eines affektiven Zustands. Denkbar sind auch andere Stimulantien wie Schreibmaschineschreiben, Hangman, Kreuzworträtsel-Lösen, das Durchführen von Intelligenztests oder Eignungstests wie dem Medizinertest.

Das Einrichtungssystem weist eine Körperinformationserfassungsvorrichtung 2 auf. Die Körperinformationserfassungsvorrichtung 2 ist eine Vorrichtung zur Erfassung physiologischer Information über das Trainingssubjekt. Im Ausführungsbeispiel handelt es sich bei der Körperinformationserfassungsvorrichtung 2 um ein Empatica E4^{®} Armbandgerät, das physiologische Signale wie elektrodermale Aktivität, Herzfrequenz und Herzfrequenzvarianz erfasst.

Das Einrichtungssystem weist ferner eine Trainingsdatenspeichervorrichtung 3 auf. Die Trainingsdatenspeichervorrichtung 3 ist eine elektronische Datenverarbeitungsvorrichtung, die mittels elektronischer Datenkommunikation, beispielsweise Bluetooth oder WLAN derart mit der Affektzustandserzeugungsvorrichtung 1 und der Körperinformationserfassungsvorrichtung 2 verbunden ist, dass Informationen von der Affektzustandserzeugungsvorrichtung 2 und der Körperinformationserfassungsvorrichtung 2 an die Trainingsdatenspeichervorrichtung 3 übermittelt werden können.

Das Einrichtungssystem weist ferner eine Trainingsvorrichtung 4 auf. Die Trainingsvorrichtung 4 ist eine elektronische Datenverarbeitungsvorrichtung, die eine CNN-Architektur (Convolutional Neural Network) implementiert. Das CNN besteht aus vier Faltungsschichten (32 Filter, Kernelgröße 3), die über Max-Pooling-Schichten verbunden sind. Nach den Konvolutionen führt eine vollständig verbundene Schicht (fully connected layer, 32 Neuronen) zu einer abschließenden dichten Schicht (dense layer) mit der Anzahl der Neuronen entsprechend der Anzahl der Klassen der Klassifikationsaufgabe und einer Softmax-Aktivierung. Die Trainingsvorrichtung 4 ist mittels elektronischer Datenkommunikation, beispielsweise Ethernet mit der Trainingsdatenspeichervorrichtung 3 derart verbunden, dass Trainingsdaten von der Trainingsdatenspeichervorrichtung 3 an die Trainingsvorrichtung 4 übermittelt werden können.

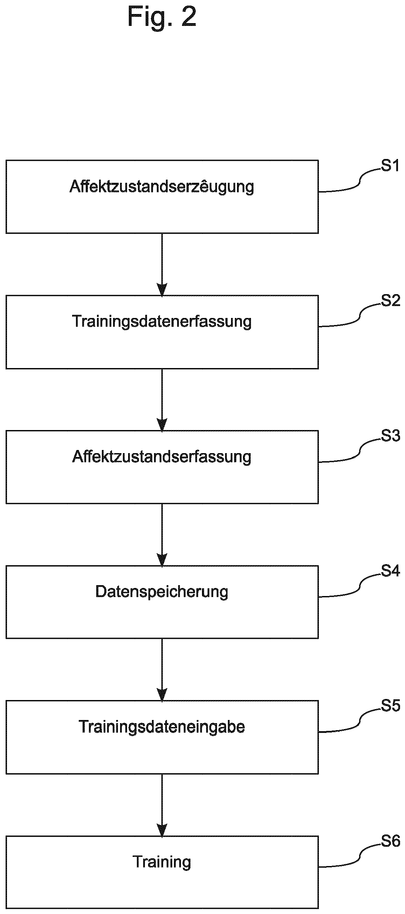

Anhand von Figur 2 wird ein Verfahren zur Einrichtung eines Systems zur Erkennung affektiver Zustände eines Subjekts erläutert.

In einem Affektzustandserzeugungsschritt S1 wird ein Trainingssubjekt in einen definierten affektiven Zustand versetzt. Hierfür wird das Trainingssubjekt mit der Aufgabe betraut, mit der Affektzustandserzeugungsvorrichtung 1 das modifizierte Spiel Tetris^{®} in einer vorgegebenen Schwierigkeitsstufe zu spielen.

Während des Spiels werden in einem Trainingsdatenerfassungsschritt S2 mittels der Körperinformationserfassungsvorrichtung 2 die physiologischen Informationen elektrodermale Aktivität, Herzfrequenz und Herzfrequenzvarianz erfasst und an die Trainingsdatenspeichervorrichtung 3 übermittelt.

In einem Zustandsermittlungsschritt S3 wird der der Schwierigkeitsstufe zugeordnete affektive Zustand erfasst und an die Trainingsdatenspeichervorrichtung 3 übermittelt.

In einem Trainingsdatenspeicherschritt werden in der Trainingsdatenspeichervorrichtung 3 die in dem Trainingsdatenerfassungsschritt S2 ermittelten physiologischen Informationen unter Zuordnung des im Zustandsermittlungsschritt S3 ermittelten affektiven Zustands gespeichert.

Die Schritte S1 bis S4 können für verschiedene Trainingssubjekte oder verschiedene affektive Zustände wiederholt werden.

In einem Trainingsdateneingabeschritt S5 werden die in der Trainingsdatenspeichervorrichtung 3 gespeicherten physiologischen Informationen und die diesen zugeordneten affektiven Zuständen dem CNN der Trainingsvorrichtung 4 zugeführt.

In einem Trainingsschritt S6 wird das CNN der Trainingsvorrichtung 4 mittels der physiologischen Information und der diesen zugeordneten affektiven Zustände trainiert.

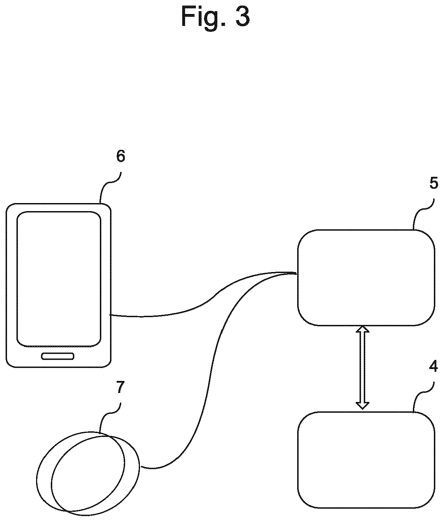

Anhand von Figur 3 wird ein System zur Erkennung affektiver Zustände eines Subjekts erläutert.

Das Erkennungssystem weist die Trainingsvorrichtung 4 des Einrichtungssystems auf, das gemäß dem Verfahren aus Fig. 2 trainiert wurde.

Das Erkennungssystem weist ferner eine Interaktionsvorrichtung 5 auf. Die Interaktionsvorrichtung 5 ist eine elektronische Datenverarbeitungsvorrichtung und stellt eine Benutzungsschnittstelle bereit, über die ein Benutzer physiologische Informationen an die Interaktionsvorrichtung 5 übermitteln kann. Ein Benutzer kann beispielsweise eine Videodatei eines Gesichts eines Subjekts oder eine Audiodatei einer Stimme eines Subjekts oder andere physiologische Informationen wie solche eines Fitnesstrackers übermitteln. Die Interaktionsvorrichtung 5 ist derart mit der Trainingsvorrichtung 4 verbunden, dass die physiologischen Informationen von der Interaktionsvorrichtung 5 an die Trainingsvorrichtung 4 übermittelt werden können und korrelierende affektive Zustände durch die Trainingsvorrichtung 4 ermittelt und an die Interaktionsvorrichtung 5 übermittelt werden können. Die Interaktionsvorrichtung 5 ist eingerichtet, dem Benutzer Informationen zum ermittelten affektiven Zustand anzuzeigen oder anderweitig zu übermitteln, beispielsweise durch Übermittlung einer Datei.

Alternativ ist die Interaktionsvorrichtung 5 eingerichtet, einem Benutzer Inhalte oder Aufgaben zu präsentieren, die dieser konsumieren oder ausführen soll. Hierbei können physiologische Informationen des Benutzers als Subjekt während der Konsumierung oder Ausführung erfasst und durch die Trainingsvorrichtung 4 ausgewertet werden.

Die Trainingsvorrichtung 4 ist so konfiguriert, dass sie mehrere Module aufweist. Ein visuelles Modul ist eingerichtet, aus visuellen Informationen, insbesondere Bewegtbildinformationen eines menschlichen Gesichts affektive Zustände zu klassifizieren. Ein physiologisches Modul ist eingerichtet, aus physiologischen Signalinformationen, insbesondere elektrodermale Aktivitätsinformationen, Herzfrequenzinformationen und Herzfrequenzvarianzinformationen affektive Zustände zu klassifizieren. Ein Akustisches Modul ist eingerichtet, aus akustischen Informationen, insbesondere der Aufnahme einer menschlichen Stimme als physiologische Information den affektive Zustände zu ermittelt.

Die Interaktionsvorrichtung 5 ist für Benutzer über ein Weitverkehrsnetz, beispielsweise dem Internet zugänglich. Physiologische Informationen können durch den Benutzer beispielsweise durch Sensoren seines Endgerätes erfasst und an die Interaktionsvorrichtung 5 übermittelt werden. Der Benutzer kann beispielsweise an einem Smartphone 6 bei der Lösung einer ihm gestellten Aufgabe über die Kamera des Smartphones ein Bewegtbild seines Gesichtes an die Interaktionsvorrichtung 5 übermitteln. Dieses Bewegtbild ermöglicht über Hautverfärbung die Ermittlung von Herzfrequenz, Herzfrequenzvarianz und elektrodermale Aktivität. Diese Informationen sind durch das visuelle Modul der Trainingsvorrichtung 4 auswertbar.

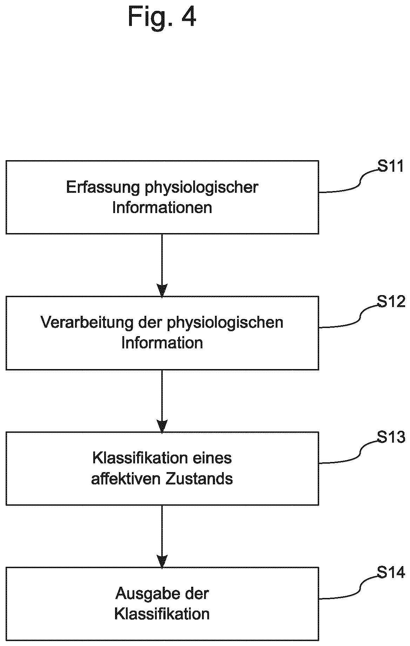

Anhand von Fig. 4 wird ein Verfahren zur Erkennung affektiver Zustände eines Subjekts erläutert.

In einem Erfassungsschritt S11 werden physiologische Informationen des Subjekts erfasst. Dies kann beispielsweise durch Erfassung eines Bewegtbildes des Gesichts des Subjekts oder durch eine Erfassung unmittelbarer physiologischer Signalinformationen wie elektrodermale Aktivität, Herzfrequenz und Herzfrequenzvarianz mittels eines Empatica E4 Armbandgeräts erfolgen. Die physiologische Information wird durch die Interaktionsvorrichtung 5 erhalten und an die Trainingsvorrichtung 4 übermittelt.

In einem Verarbeitungsschritt S12 verarbeitet die Trainingsvorrichtung 4 die physiologische Information.

In einem Klassifikationsschritt S13 klassifiziert die Trainingsvorrichtung 4 einen der physiologischen Information zugeordneten affektiven Zustand.

In einem Ausgabeschritt S14 wird der klassifizierte affektive Zustand von der Trainingsvorrichtung 4 an die Interaktionsvorrichtung 5 übermittelt und durch die Interaktionsvorrichtung 5 dem Benutzer in der Bedienoberfläche angezeigt oder zur Übermittlung, beispielsweise als Datei angeboten.

Die Erfindung ist nicht auf die oben beschriebenen Ausführungs-beispiele beschränkt. Für den Fachmann offensichtlich sind Ab-wandlungen der Ausführungsbeispiele möglich, ohne aus dem Schutzbereich der Erfindung zu fallen, wie er durch die Ansprüche definiert wird.

So ist im Ausführungsbeispiel die Affektzustandserzeugungsvorrichtung 2 eine elektronische Datenverarbeitungsvorrichtung mit einer modifizierten Variante des Spiels Tetris^{®}. Alternativ können auf der Affektzustandserzeugungsvorrichtung 2 andere Spiele oder Applikationen installiert sein, die ein Trainingssubjekt in einen affektiven Zustand versetzen können, der an eine Datenspeichervorrichtung übermittelbar ist.

Alternativ kann jede andere Vorrichtung oder Umgebung als Affektzustandserzeugungsvorrichtung verwendet werden, die ein Trainingssubjekt in einen affektiven Zustand versetzen kann, der mindestens mittelbar an eine Datenspeichervorrichtung übermittelbar ist.

Im Ausführungsbeispiel ist die Körperinformationserfassungsvorrichtung 2 ein Empatica E4^{®} Armbandgerät. Alternativ kann jede andere Vorrichtung verwendet werden, die physiologische Informationen eines Trainingssubjekts erfassen und weiterleiten kann. Dies kann insbesondere eine Kamera sein, die geeignet ist, Bewegtbildinformation eines Gesichts des Trainingssubjekts zu erfassen und zu übermitteln.

Im Ausführungsbeispiel sind die Trainingsdatenspeichervorrichtung 3, die Trainingsvorrichtung 4 und die Interaktionsvorrichtung 5 separate Vorrichtungen. Alternativ können die Funktionen dieser Vorrichtungen jedoch jeweils auf mehrere Vorrichtungen verteilt sein oder in unterschiedlichen Kombinationen in einer Vorrichtung kombiniert werden.

### Definitionen:

Die folgenden aufgelisteten Terme werden in der Beschreibung und in den Ansprüchen in der hier angegebenen Bedeutung verwendet:

| | |
|---|---|
| Subjekt: | menschlicher Teilnehmer, dessen affektiver Zustand Gegenstand einer Zustandsbestimmung ist oder sein soll. |
| Trainingssubjekt | menschlicher Teilnehmer, dessen affektiver Zustand Gegenstand des Trainings eines selbstlernenden Systems ist. |
| Softmax-Aktivierung | eine spezialisierte Aktivierungsfunktion für Klassifikationsnetze mit Einer-von-N-Kodierung. Sie erstellt ein normalisiertes Exponential, d. h. die Ausgaben summieren sich zu 1. In Kombination mit der Kreuzentropie-Fehlerfunktion, ermöglichte sie eine Modifizierung von Mehrschicht-Perzeptron-Netzen zur Schätzung von Klassenwahrscheinlichkeiten. |
| physiologische Information: | |
| | Information, die eine oder mehrere, messbare Eigenschaften des Körpers eines Subjekts oder Trainingssubjekts betrifft, wie beispielsweise Herzfrequenz und Herzfrequenzvarianz. Im Sinne dieser Lehre stellen auch Stimmoduluation, Mimik, Körperhaltung und Bewegungsmuster solche physiologische Informationen dar. |
| affektiver Zustand | ein einer Emotion oder einer Gemütslage zugeordneter Zustand eines Subjektes, der im Valenz-/Emotionsraum mit den beiden Achsen Valenz und Arousal zugeordet werden kann und sich in physiologischen Informationen über das Subjekt niederschlägt. |
| Langeweile | ein affektiver Zustand unwohlen, unangenehmen Gefühls, das durch erzwungenes Nichtstun hervorgerufen wird oder bei einer als monoton oder unterfordernd empfundenen Tätigkeit aufkommen kann. |
| Flow | auch Funktionslust oder Tätigkeitsrausch, ist ein affektiver Zustand als beglückend erlebten Gefühls völliger Vertiefung (Konzentration) und restlosen Aufgehens in einer Tätigkeit (Absorption), der sich in überdurchschnittlich hoher Produktivität äußert. |
| Stress | ein affektiver Zustand, der durch spezifische äußere Reize (Stressoren) hervorgerufen wird und zu einer körperlichen oder geistigen Überforderung führt. |
| [elektronische] Datenverarbeitungsvorrichtung: | |
| | Vorrichtung, die dazu eingerichtet ist, in elektronischer Form Daten sendend und empfangend zu übermitteln, zu speichern oder zu verarbeiten. |
| und | Junktor in der Bedeutung der logischen Konjunktion (mathematisches UND) |
| oder | Junktor in der Bedeutung der logischen Adjunktion (mathematisches ODER, oft auch "und/oder") |
| entweder ... oder | Junktor in der Bedeutung der logischen Kontravalenz (mathematisches Exklusiv-ODER) |

## Patentansprüche

1. Verfahren zur Einrichtung eines Systems zur Erkennung affektiver Zustände eines Subjekts, das die Schritte aufweist:
a. Bereitstellen einer Umgebung, die eingerichtet ist, ein Trainingssubjekt zu beobachten und in eine Gruppe von affektiven Zuständen zu versetzen, wobei die Gruppe von affektiven Zuständen mindestens einen ersten affektiven Zustand und einen zweiten affektiven Zustand umfasst und der erste affektive Zustand und der zweite affektive Zustand unterschiedlich sind,
b. Bereitstellen eines Systems (1, 2, 3, 4) zur Einrichtung eines Systems zur Erkennung affektiver Zustände des Subjekts, wobei das System ein selbstlernendes System ist und
eine erste Eingabevorrichtung zur Eingabe von physiologischen Informationen über ein Trainingssubjekts und
eine zweite Eingabevorrichtung zur Eingabe oder automatischen Erfassung des Vorliegens eines affektiven Zustands des Trainingssubjekts aufweist,
c. Versetzen des Trainingssubjektes in einen affektiven Zustand aus der Gruppe affektiver Zustände,
d. Erfassung der physiologischer Informationen über das Trainingssubjekt,
e. Ermittlung des affektiven Zustands,
f. Speicherung der erfassten physiologischen Information über das Trainingssubjekt unter Zuordnung des ermittelten affektiven Zustands,
g. Eingabe der erfassten physiologischen Information in die erste Eingabevorrichtung,
h. Eingabe des ermittelten affektiven Zustands in die zweite Eingabevorrichtung, und
i. Verarbeitung der Eingabe in der ersten Eingabevorrichtung und in der zweiten Eingabevorrichtung zum Training des Systems zur Erkennung affektiver Zustände des Subjekts,
wobei die Gruppe affektiver Zustände den affektiven Zustand Flow umfasst und
das lernende System ein neuronales Netz, ein faltendes neuronales Netz (CNN) oder ein rekurrentes neuronales Netz (RNN) aufweist.

2. Das Verfahren zur Einrichtung eines Systems zur Erkennung affektiver Zustände eines Subjekts nach dem vorhergehenden Anspruch, wobei die Schritte c. bis i. für mehrere Trainingssubjekte oder für mehrere affektive Zustände widerholt werden.

3. Das Verfahren zur Einrichtung eines Systems zur Erkennung affektiver Zustände eines Subjekts nach einem der vorhergehenden Ansprüche, wobei die Gruppe affektiver Zustände ferner den affektiven Zustand Langeweile und den affektiven Zustand Stress umfasst.

4. Das Verfahren zur Einrichtung eines Systems zur Erkennung affektiver Zustände eines Subjekts nach einem der vorhergehenden Ansprüche, wobei die physiologischen Informationen visuelle Informationen, physiologische Signalinformationen oder akustische Informationen sind.

5. Das Verfahren zur Einrichtung eines Systems zur Erkennung affektiver Zustände eines Subjekts nach dem vorhergehenden Anspruch, wobei
die visuellen Informationen Einzelbildinformationen oder Bewegtbildinformation eines menschlichen Gesichts sind,
die physiologischen Signalinformationen elektrodermale Aktivitätsinformationen, Herzfrequenzinformationen und Herzfrequenzvarianzinformationen sind, oder
die akustischen Informationen die Aufnahme einer menschlichen Stimme ist.

6. Das Verfahren zur Einrichtung eines Systems zur Erkennung affektiver Zustände eines Subjekts nach einem der vorhergehenden Ansprüche, wobei die Umgebung, die eingerichtet ist, das Trainingssubjekt in eine Gruppe von affektiven Zuständen zu versetzen, eine Aufgabenstellvorrichtung, beispielsweise eine elektronische Datenverarbeitungsvorrichtung (1) umfasst, mittels der das Trainingssubjekt eine Aufgabe gestellt wird, beispielsweise das Spiel Tetris^{®} zu spielen, wobei die Aufgabe so eingerichtet ist, dass sie mindestens so viele Schwierigkeitsstufen aufweist, wie es affektive Zustände in der Gruppe der affektiven Zustände gibt, und es eine surjektive, vorzugsweise bijektive Zuordnung von Schwierigkeitsstufen zu den affektiven Zuständen in der Form gibt, dass das Trainingssubjekt beim Lösen der Aufgabe in einer der Schwierigkeitsstufen in einen bestimmten affektiven Zustand versetzt wird.

7. Das Verfahren zur Einrichtung eines Systems zur Erkennung affektiver Zustände eines Subjekts nach dem vorhergehenden Anspruch, wobei das Spiel Tetris^{®} die Schwierigkeitsstufen leicht, mittel und schwer aufweist, wobei die Schwierigkeitsstufen so eingerichtet sind, dass sie das Trainingssubjekt beim Spielen der jeweiligen Schwierigkeitsstufe in den Zustand Langeweile, Flow, bzw. Stress versetzen.

8. Das Verfahren zur Einrichtung eines Systems zur Erkennung affektiver Zustände eines Subjekts nach dem vorhergehenden Anspruch, wobei
das selbstlernende System ein faltendes neuronales Netz aufweist, das aus vier Faltungsschichten mit 32 Filtern und einer Kernelgröße von 3 besteht,
die Schichten über Max-Pooling-Schichten verbunden sind,
nach den Konvolutionen eine vollständig verbundene Schicht (fully connected layer) mit 32 Neuronen zu einer abschließenden dichten Schicht (dense layer) führt, und
die abschließende dichte Schicht eine Anzahl von Neuronen entsprechend der Anzahl der Klassen der Klassifikationsaufgabe und einer Softmax-Aktivierung aufweist.

## Claims

1. A method of setting up a system for recognizing affective states of a subject, comprising the steps of:
a. providing an environment configured to observe a training subject and place the training subject in a set of affective states, wherein the set of affective states comprises at least a first affective state and a second affective state, and the first affective state and the second affective state are different,
b. providing a system (1, 2, 3, 4) for setting up an affective state recognition system, the system being a self-learning system and comprising
a first input device for inputting physiological information about a training subject and
a second input device for inputting or automatically recognizing the presence of an affective state of the training subject,
c. putting the training subject into an affective state from the set of affective states,
d. acquiring the physiological information about the training subject,
e. determining the affective state,
f. storing the acquired physiological information about the training subject with assignment of the determined affective state,
g. inputting the sensed physiological information into the first input device,
h. inputting the determined affective state to the second input device, and
i. processing the input in the first input device and in the second input device to train the system for recognizing affective states,
wherein the set of affective states comprises the affective state flow and the learning system comprises a neural network, a convolutional neural network (CNN), or a recurrent neural network (RNN).

2. The method of setting up a system for recognizing affective states of a subject according to the preceding claim, wherein steps c. to i. are repeated for multiple training subjects or for multiple affective states.

3. The method of setting up a system for recognizing affective states of a subject according to any one of the preceding claims, wherein the set of affective states comprises the affective state boredom and the affective state stress.

4. The method of setting up a system for recognizing affective states of a subject according to any one of the preceding claims, wherein the physiological information is visual information, physiological signal information, or acoustic information.

5. The method of setting up a system for recognizing affective states of a subject according to the preceding claim, wherein
the visual information is still image information or moving image information of a human face,
the physiological signal information is electrodermal activity information, heart rate information, and heart rate variance information, or
the acoustic information is the recording of a human voice.

6. The method of setting up a system for recognizing affective states of a subject according to any one of the preceding claims, wherein the environment configured to put the training subject into a set of affective states comprises a task setting device, for example an electronic data processing device (1), by means of which the training subject is set a task, for example to play the game Tetris^{®}, wherein the task is arranged to have at least as many difficulty levels as there are affective states in the set of affective states, and there is a surjective, preferably bijective, assignment of difficulty levels to the affective states in the form that the training subject is put into a certain affective state when solving the task in one of the difficulty levels.

7. The method of setting up a system for recognizing affective states of a subject according to the preceding claim, wherein the game Tetris^{®} has difficulty levels of easy, medium, and hard, the difficulty levels being arranged to place the training subject in the state of boredom, flow, and stress, respectively, when playing at the respective difficulty level.

8. The method of setting up a system for recognizing affective states of a subject according to the preceding claim, wherein
the self-learning system comprises a convolutional neural network consisting of four convolutional layers with 32 filters and a kernel size of 3,
the layers being connected via max-pooling layers,
after the convolutions, a fully connected layer with 32 neurons leads to a final dense layer, and
the final dense layer has a number of neurons corresponding to the number of classes of the classification task and comprises a Softmax activation.

## Revendications

1. Procédé de mise en oeuvre d'un système de reconnaissance d'états affectifs d'un sujet, comportant les étapes suivantes :
a. fournir un environnement apte à observer un sujet d'entraînement et à le placer dans un groupe d'états affectifs, le groupe d'états affectifs comprenant au moins un premier état affectif et un deuxième état affectif, le premier état affectif et le deuxième état affectif étant différents,
b. fournir un système (1, 2, 3, 4) de mise en oeuvre d'un système de reconnaissance d'états affectifs du sujet, le système étant un système d'autoapprentissage, et comprend
un premier dispositif d'entrée pur entrer des informations physiologiques sur un sujet d'entraînement, et
un deuxième dispositif d'entrée pur entrer ou détecter automatiquement la présence d'un état affectif du sujet d'entraînement,
c. placer le sujet d'entraînement dans un état affectif du groupe d'états affectifs,
d. détecter les informations physiologiques sur le sujet d'entraînement,
e. déterminer l'état affectif,
f. mémoriser les informations physiologiques détectées sur le sujet d'entraînement en associant l'état affectif déterminé,
g. entrer les informations physiologiques détectées dans le premier dispositif d'entrée,
h. entrer l'état affectif déterminé dans le deuxième dispositif d'entrée, et
i. traiter les entrées dans le premier dispositif d'entrée et dans le deuxième dispositif d'entrée pour entraîner le système de reconnaissance d'états affectifs du sujet,
dans lequel le groupe d'états affectifs comprend l'état affectif de flow, et
le système d'apprentissage comporte un réseau neuronal, un réseau convolutif (CNN) ou un réseau neuronal récurrent (RNN).

2. Le procédé de mise en oeuvre d'un système de reconnaissance d'états affectifs d'un sujet selon la revendication précédente, dans lequel les étapes c. à i. sont répétées pour plusieurs sujets d'entraînement ou pour plusieurs états affectifs.

3. Le procédé de mise en oeuvre d'un système de reconnaissance d'états affectifs d'un sujet selon l'une des revendications précédentes, dans lequel le groupe d'états affectifs comprend en outre l'état affectif d'ennui et l'état affectif de stress.

4. Le procédé de mise en oeuvre d'un système de reconnaissance d'états affectifs d'un sujet selon l'une des revendications précédentes, dans lequel les informations physiologiques sont des informations visuelles, des informations de signal physiologiques ou des informations acoustiques.

5. Le procédé de mise en oeuvre d'un système de reconnaissance d'états affectifs d'un sujet selon la revendication précédente, dans lequel
les informations visuelles sont des informations d'image ou des informations d'image animée d'un visage humain,
les informations de signal physiologiques sont des informations d'activité électrodermique, des informations de fréquence cardiaque et des informations de variance de fréquence cardiaque, ou
les informations acoustiques sont des informations d'enregistrement d'une voix humaine.

6. Le procédé de mise en oeuvre d'un système de reconnaissance d'états affectifs d'un sujet selon l'une des revendications précédentes, dans lequel l'environnement qui est adapté pour placer le sujet d'entraînement dans un groupe d'états affectifs comprend un dispositif de pose de tâches, par example un dispositif électronique de traitement de données (1), au moyen duquel le sujet d'entraînement est chargé d'une tâche, par example de jouer un jeu de Tetris^{®}, la tâche étant ainsi adaptée pour qu'elle présente au moins autant de difficultés, qu'il y a d'états affectifs dans le groupe d'états affectifs, et qu'il y a une association surjective, de préférence bijective, des difficultés à des états affectifs de tel sorte que le sujet d'entraînement est placé dans un état affectif déterminé lors de la résolution de la tâche dans l'une des difficultés.

7. Le procédé de mise en oeuvre d'un système de reconnaissance d'états affectifs d'un sujet selon la revendication précédente, dans lequel le jeu Tetris^{®} comporte les difficultés légère, moyenne et lourde, dans lequel la difficulté est telle que le sujet d'entraînement est amené à jouer la difficulté respective dans l'état d'ennui, de flow, ou de stress.

8. Le procédé de mise en oeuvre d'un système de reconnaissance d'états affectifs d'un sujet selon la revendication précédente, dans lequel
le système d'autoapprentissage comporte un réseau convolutif, constitué de couches de convolution avec 32 filtres et d'une taille de noyau de 3,
les couches sont reliées par des couches de max-pooling,
après les convolutions, une couche entièrement reliée (fully connected layer) avec 32 neurones conduit à une couche finale dense (dense layer), et
la couche finale dense comporte un nombre de neurones correspondant au nombre de classes de la tâche de classification et une activation softmax.
